# EUROPÄISCHE PATENTSCHRIFT

(11) **EP 0 698 122 B1**
(45) Veröffentlichungstag und Bekanntmachung des Hinweises auf die Patenterteilung: **26.03.1997**
(21) Anmeldenummer: 94916131.9
(22) Anmeldetag: 17.05.1994
(51) Int. Cl.: C12Q 1/68, C12P 19/34

(54) **MITTEL ZUR KOMPLEXEN DIAGNOSTIK DER GENEXPRESSION UND VERFAHREN ZUR ANWENDUNG FÜR DIE MEDIZINISCHE DIAGNOSTIK UND DIE GENISOLIERUNG**
COMPLEX DIAGNOSTIC AGENT OF GENETIC EXPRESSION AND MEDICAL DIAGNOSIS AND GENE ISOLATION PROCESS USING SAID DIAGNOSTIC AGENT
AGENT DE DIAGNOSTIC COMPLEXE DE L'EXPRESSION GENETIQUE ET SON PROCEDE D'APPLICATION EN DIAGNOSTIC MEDICAL ET POUR ISOLER DES GENES

(30) Priorität: 18.05.1993 DE 4317414
(43) Veröffentlichungstag der Anmeldung: 28.02.1996
(73) Patentinhaber: Max-Planck-Gesellschaft zur Förderung der Wissenschaften e.V., 80539 München (DE)
(72) Erfinder: STRAUSS, Michael, D-13187 Berlin (DE); BAUER, David, D-13156 Berlin (DE)
(74) Vertreter: Baumbach, Friedrich, Dr.
(86) Internationale Anmeldenummer: DE9400568
(87) Internationale Veröffentlichungsnummer: WO9426928

(56) Entgegenhaltungen:
- EP-A- 0 531 027
- WO-A-93/18176
- DE-A- 4 317 414
- US-A- 5 104 792
- SCIENCE, Bd.257, 14. August 1992, AAAS, WASHINGTON, DC, US; Seiten 967 - 971 P. LIANG AND A.B. PARDEE 'Differential display of eucaryoric messenger RNA by means of the polymerase chain reaction' in der Anmeldung erwähnt
- CANCER REASERCH, Bd.52, Nr.24, 15. Dezember 1992, WARVERLY PRESS INC., BALTIMORE,US; Seiten 6966 - 6968 P. LIANG ET AL. 'Differential display and cloning of messenger RNA from human breast cancer versus mammary epithelial cells' in der Anmeldung erwähnt
- ANALYTICAL BIOCHEMISTRY, Bd.198, Nr.1, Oktober 1991, ACADEMIC PRESS, INC., NEW YORK, US; Seiten 86 - 91 A. LANDGRAF ET AL. 'Direct analysis of polymerase chain reaction products using enzyme-linked immunoabsorbent assay techniques'
- NUCL. ACIDS RES., Bd.21, Nr.18, 11. September 1993, IRL PRESS, OXFORD, ENGLAND; Seiten 4272 - 4280 D. BAUER ET AL. 'Identification of differentially expressed mRNA species by an improved display technique (DDRT-PCR)' in der Anmeldung erwähnt
- BIOTEC, Bd.6, Nr.2, April 1994, BIOTEC, WüRZBURG, BRD; Seiten 32 - 35 D. BAUER ET AL. 'Differential display reverse transcription PCR'

## Beschreibung

Die Erfindung betrifft ein Mittel zur komplexen Diagnostik der Genexpression und ein Verfahren zur Anwendung des Mittels. Anwendungsgebiete der Erfindung sind die Molekularbiologie, insbesondere die Untersuchung von exprimierten Genen und die Genisolierung sowie die klinische Diagnostik von krankhaften Veränderungen einschließlich Krebs.

Die Analyse des menschlichen Genoms hat in den letzten Jahren große Fortschritte gemacht. Die Ermittlung der Bausteinreihenfolge des gesamte Erbmaterials (Genoms) wird international angestrebt. Damit werden Aussagen über Genstruktur und konkrete genetische Defekte möglich, die zur Personenidentifikation angewendet werden können. Da das Gesamtgenom sehr komplex ist, wird alternativ die Ermittlung der Sequenzen nur der tatsächlich aktiven Gene angestrebt. Dies ist relativ leicht möglich durch das Anlegen einer cDNA-Bibliothek eines bestimmten Zelltyps und die anschließende Sequenzierung aller Einzelklone (PCT-Patent WO 93/353).
Die Polymerase-Ketten-Reaktion oder PCR-Technik hat andererseits die Möglichkeit geschaffen, jedes beliebige DNA-Stück oder Gen zu identifizieren und zu vermehren. Diese Technik läßt sich auf die Identifizierung exprimierter Gene, d.h. den Nachweis der Synthese einer entsprechenden mRNA, anwenden (Rappolee, D.A. et al. Science 241/1988/708). Dabei wird zunächst mRNA mit Hilfe einer Transkriptase in cDNA umgeschrieben, die dann als Matrize für die Amplifikation mit bestimmten Primern eingesetzt wird. Diese Technik wird heute bereits für die Diagnostik der Expression bestimmter Gene im Routinelabor eingesetzt. Sie ist aber nur auf bereits bekannte Gene anwendbar, da die Primer aufgrund der bekannten Gensequenz festgelegt und synthetisiert wurden.

Für die molekulare Diagnostik von Veränderungen in der Genexpression, die beispielsweise mit Krankheitsprozessen einhergehen, wäre die Verfügbarkeit einer Technik wünschenswert, die alle, also auch noch unbekannte, Genfunktionen erfassen ließe. Das bedeutet, daß ein Weg gefunden werden muß, um alle mRNA-Spezies auch ohne Kenntnis ihrer Sequenz zu identifizieren. Eine derartige Technik wurde erstmals von Liang und Pardee (Science 257/1992/967) vorgestellt. Sie basiert auf der fraktionierten Herstellung von cDNA mit Primern, die entweder 12 oder 4 Fraktionen entstehen lassen. Diese cDNA-Fraktionen werden anschließend mit mehreren kurzen Primern in unabhängigen Reaktionen amplifiziert. Die Primer sind dabei so kurz (10 Nukleotide), daß ihre Chance relativ groß ist, in vielen cDNA-Spezies Homologien zu finden. Statistisch kann dabei davon ausgegangen werden, daß die Länge der entstehenden Fragmente unterschiedlich groß ist. Wenn einen genügend große Zahl von Primern geeigneter Sequenz eingesetzt wird, sollte es möglich sein, prinzipiell alle exprimierten mRNAs als cDNA-Fragment zu amplifizieren. Die Identifizierung der Fragmente wurde von Liang und Pardee nach radioaktiver Markierung durch Elektrophorese in einem Polyacrylamid-Sequenzierungsgel durchgeführt. Diese Autoren haben ihre Methode zunächst zur Identifizierung von veränderter Genexpression in Brustkrebszellen eingesetzt (Science 257/1992/967, Cancer Research 52/1992/6966) dabei einige veränderte Banden identifiziert und anschließend die dazugehörigen Gene teilweise isoliert.

Bei der Verwendung der beschriebenen Methodik wird offensichtlich, daß die Identifizierung vieler Fragmente bzw. Banden auf einem Sequenzierungsgel möglich ist und beim Vergleich identischer PCR-Reaktionen von zwei Zelltypen auch viele Unterschiede gefunden werden können. Allerdings konnte festgestellt werden (Bauer et al., Nucleic Acids Research 21, 1993, S. 4272-4280), daß 1/3 bis 2/3 aller Fragmente tatsächlich nicht nur eine Bande, sondern 2-4 Banden produzieren, was mit der Eigenschaft der Taq-Polymerase zusammenhängt, am 3'-Ende der kopierten Stränge noch ein zusätzliches A anzuhängen. Darüberhinaus werden häufig die zwei Stränge eines Fragments aufgrund unterschiedlichen Purin-/Pyrimidin-Gehaltes separiert.

So treten veränderte Banden vielfach als Block von unmittelbar aufeinanderfolgenden Banden auf. Dadurch wird eine Komplexität des Musters vorgetäuscht, die nicht vorhanden ist.

Die von Pardee und Liang angegebenen Primer reichen darüberhinaus keinesfalls aus, um die notwendige Komplexität des Fragmentmusters zu erreichen. Bei einer angenommenen Zahl von ca. 15 000 Genen, die in einer Zelle exprimiert werden, müßten mindestens 30 000 verschiedene Fragmente produziert und sichtbar gemacht werden, um mit großer Wahrscheinlichkeit auch nahezu jede mRNA erfaßt zu haben, wobei eine größere Zahl von Genen zweimal und eine kleinere Zahl dreimal oder häufiger repräsentiert sein müßten. Darüberhinaus wäre es wichtig,, das Diagnose-Verfahren universell anwendbar zu machen. Dafür ist eine Vereinfachung und gleichzeitige Objektivierung der Bandenerfassung notwendig.

Das Ziel der Erfindung besteht darin, die Untersuchung der Genexpression zu verbessern bzw. überhaupt erst zu ermöglichen. Ihr liegt die Aufgabe zugrunde, die in Zahl und Sequenz geeigneten Oligonukleotid-Primer auszuwählen,
(2.) ein Elektrophorese-System zu schaffen, das nur eine Bande pro Fragment produziert und
(3.) alles mit einer Nachweismethode zu kombinieren, die eine Automatisierung der Methode zur Diagnostik von Veränderungen der Genexpression in kürzester Zeit ermöglicht.

Das erfindungsgemäße Mittel ist durch einen Satz von mindestens 288 Oligonukleotid-Primer-Paaren definierter Länge, gebildet durch Auswahl aus einem Vorrat von mindestens 24 5'-Oligonukleotid-Primern und 12 3'-Oligonukleotid-Primern, gekennzeichnet. Diese Oligonukleotid-Primer sind mit einem geeigneten Marker gekoppelt, der durch ein Nachweisverfahren erkannt werden kann.
Die Primer wurden aus einer Liste von 2000 zufälligen, Computer-generierten Sequenzen ausgewählt, 50 ausgewählte Sequenzen wurden experimentell geprüft, 26 davon erwiesen sich als geeignet für das erfindungsgemäße Mittel.
Die Länge der Oligonukleotid-Primer beträgt 6-13 Nukleotide, bevorzugt 10 Nukleotide, wobei die Sequenzen der 5'-Primer bevorzugt die gleiche Anzahl G+C und T+A aufweisen. Die 3'-Primer besitzen eine Reihe von T-Nukleotiden, bevorzugt 8-12, und zusätzlich am Ende in ein bis drei Positionen, bevorzugt in 2 Positionen, G-, C-, T- oder A-Nukleotide. Durch Kombination jedes der mindestens 24 5'-Primer mit jedem der (im Falle von 2 spezifischen endständigen Nukleotiden)12 3'-Primern entstehen die mindestens 288 Kombinationen gemäß der Erfindung.
Alle Primer sind markiert, wobei als Markierung radioaktiver Phosphor, ein Fluoreszenzfarbstoff, Biotin oder ein Antigen verwendet wird.
Bevorzugt eingesetzt werden die 5'-Oligonukleotid-Primer gemäß Anspruch 7. Diese Primer können erfindungsgemäß auch miteinander kombiniert werden, wobei eines der beiden eingesetzten Primer als 3'-Primer fungiert. Bei dieser Möglichkeit erhält man im Erfolgsfalle Teilsequenzen der jeweiligen cDNA, womit eine weitere Identifizierung erreicht wird.

Das erfindungsgemäße Verfahren zur Anwendung des Mittels wird folgendermaßen durchgeführt
1. Die gesamte mRNA einer zu untersuchenden Zelle wird isoliert
2. Umschreibung in cDNA entweder insgesamt oder in mehreren, bevorzugt 12, Fraktionen
3. Nachfolgende Amplifizierung durch mindestens 288 parallele und unabhängige Polymerase-Ketten-Reaktionen (PCR) mit dem ausgewählten Mittel. Eine bevorzugte Temperatur für die Durchführung der Reaktion ist 40° C.
4. Auftrennung des entstandenen Fragmentgemischs jeder Reaktion auf einer gesonderten Bahn eines Polyacrylamid-Gels, bevorzugt auf 7 Gelen mit 48 Bahnen. Für den erfindungsgemäßen Erfolg ist wesentlich, daß ein nichtdenaturiertes Gel eingesetzt wird.
5. Ablesung der Bandensignale durch Autoradiographie oder durch nichtradioaktive Nachweise (Fluoreszenzfarbstoffe, Biotin oder ein Antigen) und Vergleich mit dem Muster einer Referenzzelle.

Die PCR-Reaktionen der Referenzzelle werden mit Primern durchgeführt, die mit einem anderen Farbstoff markiert sind, als die Primer für die PCR-Reaktionen der zu untersuchenden Zelle. Die mit Primern gleicher Sequenz, aber mit verschiedenen Farbstoffen markierten Fragmente von Referenz- und zu untersuchender Zelle werden gemäß der Erfindung gemeinsam in einer Bahn eines Sequenzautomaten aufgetrennt, wobei die Unterschiede der Genexpression durch ein unterschiedliches Farbsignal in einer bestimmten Position aufgezeigt werden.

Das neue Mittel kann gleichermaßen zur Isolierung von Genen oder Gensequenzen eingesetzt werden. Dabei wird eine Bande, die aufgrund der gefundenen Unterschiede zwischen den untersuchten Zellen als Repräsentant eines differentiell regulierten Gens identifiziert wurde, isoliert und als Sonde zur Gewinnung des entsprechenden Gens einer cDNA-Bank eingesetzt. Dazu wird die Bande mit einem Skalpell aus dem Gel geschnitten, in einer PCR-Inkubation mit einem biotinylierten Nukleotid amplifiziert und gleichzeitig markiert und anschließend mit einer denaturierten cDNA-Bibliothek in Lösung hybridisiert. Das resultierende Hybrid wird an magnetische Kugeln, die mit Streptavidin bedeckt sind, gebunden und von unspezifisch gebundenem Material freigewaschen. Die spezifisch gebundene cDNA wird freigesetzt und zur Transformation von E.coli eingesetzt. Aus diesen wird die Plasmid-DNA isoliert und das cDNA-Insert sequenziert. Durch Vergleich der ermittelten Sequenz mit der Sequenz-Datenbank wird festgestellt, ob es sich um ein bekanntes oder ein noch unbekanntes Gen handelt.

Das neue Mittel und das Verfahren zu seiner Anwendung erlauben die umfassende Darstellung exprimierter Gene in Form von jeweils mindestens einer Bande.
Innerhalb einer Woche kann die Analyse für einen bestimmten Zelltyp abgeschlossen werden, wonach der automatische Vergleich mit dem Muster der exprimierten mRNAs beliebiger anderer Zelltypen durchgeführt werden kann. Für die klinische Diagnostik von krankhaften genetischen Veränderungen z.B. bei Krebserkrankungen, wird durch die Erfindung ein erheblicher Fortschritt erreicht.

Die Erfindung soll nachfolgend durch Ausführungsbeispiele näher erläutert werden:

### Ausführungsbeispiele

1. Zytoplasmatische RNA der zu untersuchenden Zellen (normale Mausleber und regenerierende Mausleber) wurde inan sich bekannter Weise präpariert. Die RNA wurde anschließend auf 12 Inkubationsansätze verteilt und unter Verwendung der von Liang und Pardee (Science 257) beschriebenen Primer dT₁₁XY einer reversen Transkriptionsreaktion in bekannter Weise unterzogen (dabei stellen X und Y alle Kombinationen der 4 Nukleotide G,A,T,C dar, mit der Ausnahme, daß in der Position X kein T verwendet wird). In jede Reaktion wurden folgende Komponenten pipettiert: 0.1-0.2 µg RNA, 2.5 µM dT₁₁XY Primer, 20 µM dNTP und 300 Einheiten MMLV Reverse Transcriptase. Die Reaktionen wurden bei 35 C für eine Dauer von 60 min inkubiert und durch 5 min Inkubation bei 95° C gestoppt. Die 12 erhaltenen cDNA-Fraktionen wurden dann spezifischen Polymerasekettenreaktionen (PCR) unterzogen:
a) Zunächst wurden aus einer Zahl von 2000 zufälligen, Computer-generierten Sequenzen einer Länge von 8-13 Nukleotiden 50 Kandidaten-Sequenzen ausgewählt, synthetisiert und als Primer für PCR Inkubationen mit jeweils 3 cDNA-Fraktionen eingesetzt. Dazu wurden folgende Reaktionen angesetzt: 2.5 µM dT₁₁XY Primer, 0.5 µM des entsprechenden 5'-Primers, 2 µM dNTPs, 2 µci (60 nM) ³³P-dATP, 10 mM Tris-HCl (pH 8.3), 50 mM KCl, 1.2 mM MgCl₂, and 1 U Taq Polymerase in einem Volumen von 20 µl. Die Amplifikation wurde in 40 Zyklen mit 30 Sekunden bei 94° C, 1 Minute bei 42°C und 30 Sekunden bei 72° C durchgeführt. Die Reaktionsgemische wurden getrocknet und anschließend in 5 µl Formamid/Farbstofflösung aufgenommen. 1,5 µl wurden auf 6% Polyacrylamid-Harnstoff-Sequenzgelen aufgetrennt. Die Gele wurden getrocknet und der Autoradiographie unterzogen. Die Autoradiogramme wurden mit einem Scanner eingelesen,und die Zahl der Banden wurde ermittelt. Dabei wurde eine Zahl von 26 Primern mit sämtlichst 10 Nukleotiden Länge ermittelt , die mit den drei getesteten cDNA-Fraktionen eine Bandenzahl zwischen 40 und 160 ergaben. Diese Primer wurden als geeignet befunden, ein repräsentatives Bandenmuster aller exprimierten RNA-Spezies zu generieren.
b) Es wurde ein Versuchsprotokoll konzipiert, das eine umfassende Darstellung aller exprimierten RNAs in kürzester Zeit und mit geringstem Aufwand erlaubte:
   - Es wurden 12 PCR-Inkubationsmixturen vorbereitet, die bis auf den 5'-Primer alle unter a) genannten Komponenten enthielten, und zwar in einem Volumen von 15 µl pro Einzelreaktion und ausreichend für 24 Reaktionen. Dabei enthielt jedes der 12 Röhrchen einen anderen 3'-Primer des Typs dT₁₁XY. Mit einer 12-Kanal-Pipette wurden jeweils 15 µl der 12 Mixturen gleichzeitig in die erste Reihe einer Mikrotiterplatte (bzw. Röhrchen in diesem Format angeordnet) pipettiert. Dieser Schritt wurde 24 Mal für 3 Mikrotiterplatten wiederholt.
   - Stammlösungen von 24 verschiedenen 5'-Primern wurden so angeordnet (3x8 Reihen), daß je 5 µl von 8 verschiedenen Primern (Nr. 1-8 aus Tabelle 1) mit einer 8-Kanal-Pipette gleichzeitig in die erste senkrechte Reihe der ersten Mikrotiterplatte pipettiert werden konnten. Dieser Schritt wurde 12 Mal für die 12 senkrechten Reihen der ersten Platte wiederholt. Danach wurden die Primer Nr. 9-16 in die 12 senkrechten Reihen der zweiten Mikrotiterplatte und anschließend die Primer Nr. 17-24 in die 12 senkrechten Reihen der dritten Mikrotiterplatte transferiert. Die 288 Reaktionsansätze wurden durch kurzes Zentrifugieren der Platten gemischt. Dann wurden die drei Platten nacheinander in einem PCR-Gerät mit Mikrotiterformat-Heizblock für 40 Zyklen wie unter a) inkubiert. Anschließend wurden die Reaktionsgemische in einer Vakuumzentrifuge mit Mikrotiterplatten-Rotor bis zur Trocknung zentrifugiert. Danach wurden alle Reaktionsgemische in Formamid aufgenommen und der Elektrophorese wie unter a) unterzogen. Dazu wurden 6 Gele mit je 48 Bahnen beschickt. Nach der Trennung wurden die Gele getrocknet und mit einem Scanner eingelesen. Für die cDNAs der normalen Mausleber wurden in den 288 Bahnen insgesamt 20160 Banden identifiziert.
c) Der Versuch von b) wurde unter ansonsten gleichen Bedingungen mit verschiedenen "annealing" -Temperaturen zwischen 34° C und 45° C durchgeführt. Dabei erwies sich 40° C als das Optimum. Es wurden dabei fast doppelt so viel Banden gefunden wie bei 42° C, wobei die Muster ihre Individualität behielten.
d) Zwecks Anwendung der Methode zur Identifizierung von Unterschieden in der Genexpression zwischen zwei unterschiedlichen Zuständen eines Zellsystems wurden die RNAs von normaler Mausleber und regenerierender Mausleber verglichen. Dabei wurde die Prozedur b) mit 40° statt 42° C in gleicher Weise für beide RNAs durchgeführt, d.h. mit zwei Sätzen von je 3 Mikrotiterplatten. Die Elektrophorese wurde in insgesamt 12 Gelen durchgeführt, wobei generell die Proben mit den gleichen Primern aus beiden Ansätzen nebeneinander liefen. Im Ergebnis dieser Analyse wurden mindestens 70 Banden identifiziert, die nur in jeweils einer der beiden Ausgangs-RNAs vorhanden waren. Zahlreiche weitere Unterschiede konnten nur vermutet werden.
e) Die Charakterisierung der unterschiedlichen Banden wurde durch Sequenzierung versucht. Dabei traten Probleme auf, die vor allem durch das Vorliegen von Einzelsträngen sowie das denaturierende Gel (Harnstoff) begründet waren. Daher wurden die Proben nach der PCR-Reaktion in wäßriger Lösung auf Polyacrylamidgele ohne Harnstoff aufgetragen, die Banden ausgeschnitten und direkt eine Sequenzierungsreaktion eingebracht. Dadurch wurde zwar die Bandenzahl auf ca. die Hälfte reduziert, aber die DNA der interessierenden Banden konnte direkt als Scheibe aus dem Polyacrylamidgel in einer Sequenzierungsreaktion inkubiert und die Sequenz ermittelt werden. Die Verwendung nichtdenaturierender Gele hat sich damit als entscheidende Voraussetzung für die Identifizierung der interessanten Gensequenzen erwiesen.
   Mit dieser Methode wurde die Gesamtanalyse der exprimierten RNAs in der Mausleber mit allen 26 5'-Primern durchgeführt. Dabei wurden insgesamt ca. 38 000 Banden identifiziert, die sich statistisch auf die Primerpaaare verteilen (Tab. 1).

2. Die Methode sollte zu einer generell einsetzbaren und automatisierten Analytik ausgebaut werden, die ein zweifelsfreies Identifizieren von Unterschieden in der Genexpression zwischen zwei oder auch mehr Zuständen erlaubt, und dies möglichst nicht nur qualitativ sondern auch quantitativ. Unter 1d) wurde bereits festgestellt, daß das Nebeneinanderlaufen der zu vergleichenden Proben nicht in jedem Falle die eindeutige Identifizierung von Unterschieden ermöglicht. Daher wurde die Methodik auf den automatischen Sequenzanalysator 373A adaptiert, der bis zu vier farbmarkierte Proben in einer Bahn vergleichen kann. Dazu wurden alle 26 5'-Primer mit den vier Farbstoffen TAMRA, ROX, FAM und JOE (entsprechend Protokoll der Fa. Applied Biosystems) markiert. Die jeweils 12 cDNA-Fraktionen von normaler Leber und von drei verschiedenen Zeitpunkten der Regeneration wurden entsprechend Beispiel 1b+c amplifiziert, wobei für jede der 4 Ausgangs-RNAs eine andere Farbmarkierung gewählt wurde. Die Konzentration der markierten 5'-Primer war 0.5 µM und die dNTP-Konzentration wurde auf 100 µM erhöht. Die PCR wurde ansonsten wie unter 1b beschrieben durchgeführt. Die Trennung wurde im denaturierenden Gel im 373A durchgeführt,und die Farbmarkierung wurde aufgezeichnet.

**Tabelle 1:**

| Anzahl der Banden im nichtdenaturierenden Polyacryamidgel von cDNA-Fraktionen, die duch reverse Transkription von Mausleber-RNA mit den 12 Primern T₁₁VN erhalten wurden. Die 312 PCR-Inkubationen wurden mit den in der Tabelle angegebenen Primer-Paaren durchgeführt. (Die 5'-Primer 1-26 entsprechen den in den Ansprüchen angegebenen Primern 1-26. | | | | | | | | | | | | | | |
|---|---|---|---|---|---|---|---|---|---|---|---|---|---|---|
| T₁₁ | CA | CG | CT | CC | GA | GG | GT | GC | AA | AG | AT | AC | Σ | x |
| Primer | 1 | 2 | 3 | 4 | 5 | 6 | 7 | 8 | 9 | 10 | 11 | 12 | | |
| 1 | 92 | 188 | 73 | 137 | 186 | 182 | 67 | 189 | 121 | 142 | 129 | 147 | 1653 | 138 |
| 2 | 148 | 66 | 131 | 82 | 156 | 150 | 176 | 126 | 103 | 179 | 154 | 125 | 1596 | 133 |
| 3 | 81 | 172 | 172 | 121 | 89 | 82 | 135 | 131 | 114 | 97 | 145 | 124 | 1463 | 122 |
| 4 | 105 | 126 | 99 | 71 | 118 | 107 | 96 | 130 | 67 | 170 | 124 | 174 | 1387 | 116 |
| 5 | 94 | 182 | 104 | 157 | 101 | 91 | 70 | 91 | 115 | 89 | 148 | 103 | 1345 | 112 |
| 6 | 125 | 137 | 111 | 95 | 161 | 112 | 98 | 121 | 65 | 64 | 124 | 152 | 1365 | 114 |
| 7 | 62 | 93 | 129 | 60 | 169 | 146 | 176 | 64 | 104 | 175 | 123 | 156 | 1457 | 121 |
| 8 | 120 | 133 | 93 | 94 | 110 | 158 | 188 | 102 | 151 | 108 | 173 | 60 | 1490 | 124 |
| 9 | 183 | 157 | 124 | 148 | 173 | 167 | 180 | 167 | 76 | 146 | 90 | 87 | 1698 | 142 |
| 10 | 112 | 118 | 183 | 174 | 101 | 73 | 75 | 104 | 165 | 110 | 136 | 111 | 1462 | 122 |
| 11 | 98 | 180 | 174 | 159 | 140 | 139 | 123 | 101 | 147 | 73 | 176 | 138 | 1648 | 137 |
| 12 | 111 | 129 | 164 | 81 | 126 | 135 | 133 | 89 | 180 | 96 | 152 | 141 | 1537 | 128 |
| 13 | 82 | 139 | 149 | 135 | 124 | 146 | 71 | 73 | 136 | 153 | 86 | 90 | 1384 | 115 |
| 14 | 73 | 146 | 180 | 93 | 90 | 134 | 167 | 80 | 160 | 70 | 72 | 126 | 1391 | 116 |
| 15 | 174 | 179 | 72 | 105 | 138 | 153 | 83 | 88 | 130 | 130 | 104 | 87 | 1443 | 120 |
| 16 | 77 | 109 | 145 | 162 | 67 | 127 | 164 | 135 | 132 | 108 | 104 | 120 | 1450 | 121 |
| 17 | 65 | 105 | 135 | 136 | 129 | 170 | 151 | 85 | 130 | 106 | 159 | 178 | 1549 | 129 |
| 18 | 121 | 185 | 74 | 134 | 117 | 122 | 100 | 66 | 123 | 72 | 74 | 110 | 1298 | 108 |
| 19 | 165 | 73 | 80 | 179 | 114 | 169 | 91 | 134 | 65 | 122 | 75 | 121 | 1388 | 116 |
| 20 | 165 | 141 | 84 | 69 | 71 | 170 | 62 | 73 | 87 | 68 | 101 | 135 | 1226 | 102 |
| 21 | 80 | 137 | 156 | 120 | 94 | 179 | 66 | 126 | 161 | 131 | 188 | 89 | 1527 | 127 |
| 22 | 68 | 127 | 121 | 182 | 115 | 85 | 137 | 166 | 107 | 127 | 108 | 181 | 1524 | 127 |
| 23 | 156 | 141 | 131 | 78 | 164 | 183 | 92 | 157 | 123 | 95 | 138 | 84 | 1542 | 129 |
| 24 | 165 | 62 | 89 | 163 | 107 | 99 | 144 | 93 | 145 | 123 | 138 | 63 | 1391 | 116 |
| 25 | 82 | 185 | 187 | 71 | 114 | 184 | 140 | 103 | 142 | 101 | 116 | 170 | 1595 | 133 |
| 26 | 81 | 87 | 96 | 153 | 70 | 106 | 177 | 83 | 81 | 119 | 160 | 77 | 1290 | 108 |
| Σ 2885 3497 3152 3159 3144 3569 3167 2877 3130 2974 3297 3149 x 111 135 121 122 121 137 122 111 120 114 127 121 x = 122 | | | | | | | | | | | | | | |

## Patentansprüche

1. Mittel zur komplexen Diagnostik der Genexpression, gekennzeichnet dadurch, daß es aus mindestens 288 Oligonukleotid-Primer-Paaren besteht, welche jeweils aus einem Vorrat von mindestens 24 5'-Oligonukleotid-Primern, die eine gleiche Anzahl von G+C und A+T aufweisen, und mindestens 12 3'-Oligonukleotid-Primern, die eine Reihe von T-Nukleotiden, bevorzugt 8-12, und zusätzlich am 3'-Ende in 1-3 Positionen alle möglichen Kombinationen der 4 Nukleotide aufweisen, gebildet werden, die mit einem geeigneten Marker gekoppelt sind, der durch ein Nachweisverfahren erkannt werden kann.

2. Mittel zur komplexen Diagnostik der Genexpression nach Anspruch 1, gekennzeichnet dadurch, daß alle 5'- oder alle 3'-Primer markiert sind.

3. Mittel zur komplexen Diagnostik der Genexpression nach Anspruch 1-2, gekennzeichnet dadurch, daß als Markierung radioaktiver Phosphor, ein Fluoreszenzfarbstoff, Biotin oder ein Antigen verwendet wird.

4. Mittel zur komplexen Diagnostik der Genexpression nach Anspruch 1-3, gekennzeichnet dadurch, daß die 5'-Oligonukleotid-Primer folgende Sequenzen aufweisen:

1. 5′ T A C A A C G A G G 3′

2.    T G G A T T G G T C
3.    C T T T C T A C C C
4.    T T T T G G C T C C
5.    G G A A C C A A T C
6.    A A A C T C C G T C
7.    T C G A T A C A G G
8.    T G G T A A A G G G
9.    T C G G T C A T A G
10.   G G T A C T A A G G
11.   T A C C T A A G C G
12.   C T G C T T G A T G
13.   G T T T T C G C A G
14.   G A T C A A G T C C
15.   G A T C C A G T A C
16.   G A T C A C G T A C
17.   G A T C T G A C A C
18.   G A T C T C A G A C
19.   G A T C A T A G C C
20.   G A T C A A T C G C
21.   G A T C T A A C C G
22.   G A T C G C A T T G
23.   G A T C T G A C T G
24.   G A T C A T G G T C
25.   G A T C A T A G C G
26.   G A T C T A A G G C

5. Mittel zur komplexen Diagnostik der Genexpression nach Anspruch 1 und 4, gekennzeichnet dadurch, daß die 5'-Oligonukleotid-Primer nach Anspruch 4 als 3'-Oligonukleotid-Primer eingesetzt werden.

6. Verfahren zur Anwendung des Mittels nach Anspruch 1-5, gekennzeichnet dadurch, daß die gesamte RNA einer Zelle in cDNA umgeschrieben, diese anschließend mit dem Mittel in einer bestimmten Zahl, bevorzugt 288-360, paralleler und unabhängiger Polymerase-Ketten-Reaktionen (PCR) amplifiziert, das entstehende Fragmentgemisch jeder Reaktion auf einer Bahn eines nichtdenaturierten Polyacrylamid-Gels aufgetrennt, das Bandenmuster registriert und mit einem Standard verglichen wird, gegebenenfalls die DNA aus Banden isoliert und als Sonde zur Genisolierunq verwendet wird.

7. Verfahren nach Anspruch 6, gekennzeichnet dadurch, daß jede PCR-Reaktion, bevorzugt eine Gesamtzahl von 312, auf der entsprechenden Zahl von Polyacrylamidgelen, bevorzugt 7 Gelen mit 48 Bahnen, getrennt, die Bandensignale durch Autoradiographie oder entsprechende nichtradioaktive Nachweise sichtbar gemacht, mit einem Scanner eingelesen und mit dem Muster von einer Referenzzelle verglichen werden.

8. Verfahren nach Anspruch 6 , gekennzeichnet dadurch, daß sämtliche PCR-Reaktionen der Referenzzelle mit Primern durchgeführt werden, die mit einem bestimmten Fluoreszenz-Farbstoff markiert sind und alle PCR-Reaktionen der zu untersuchenden Zelle mit Primern durchgeführt werden, die mit einem anderen Fluoreszenz-Farbstoff markiert sind.

9. Verfahren nach Anspruch 6 und 8, gekennzeichnet dadurch, daß jeweils die mit Primern gleicher Sequenz, aber verschiedenen Farbstoffen markierten Fragmente von Referenz- und zu untersuchender Zelle gemeinsam in einer Bahn eines Sequenzautomaten mit eingebautem Farbdetektionssystem aufgetrennt werden und die Unterschiede in der Genexpression durch unterschiedliches Farbsignal in einer bestimmten Position angezeigt werden.

10. Verfahren nach Anspruch 6, gekennzeichnet dadurch, daß eine Bande aus dem Gel ausgeschnitten, ihre DNA durch PCR amplifiziert und gleichzeitig mit Biotin markiert, anschließend mit einer denaturierten cDNA-Bibliothek hybridisiert und an Streptavidin-Magnetkugeln gebunden wird, und die spezifisch gebundene cDNA nach Waschung der Kugeln, Fluierung, Bakterientransformation und Plasmidpräparation sequenziert und als bekanntes oder unbekanntes Gen identifiziert wird.

11. Verfahren nach Anspruch 6, 8 und 9, gekennzeichnet dadurch, daß die Anlagerung ("annealing") der Primer in der PCR-Reaktion bei 40° C erfolgt.

## Claims

1. A tool for complex diagnostic of gene expression, wherein said tool consists of at least 288 oligonucleotide primer pairs, which are linked with an appropriate detectable marker, composed of a pool of at least 24 5'- oligonucleotide-primers, which have the same G+C and A+T content and, at least 12 3'-oligonucleotide primers, which have a number oligo(dT)-nucleotides, preferentially 8 - 12, and additionally all possible combinations of the 4 nucleotides in 1 -3 postions at the 3'-end.

2. The tool of claim 1, wherein said 5'- and 3'-primers are labeled.

3. The tool of claim 1-2, wherein said marker is radioactive phosphorous, a fluorescence dye, biotin or an antigen.

4. The tool of claim 1-3, wherein said 5'-oligonucleotide primers have the following sequences:

1. 5´ TACAACGAGG 3´
2. TGGATTGGTC
3. CTTTCTACCC
4. TTTTGGCTCC
5. GGAACCAATC
6. AAACTCCGTC
7. TCGATACAGG
8. TGGTAAAGGG
9. TCGGTCATAG
10. GGTACTAAGG
11. TACCTAAGCG
12. CTGCTTGATG
13. GTTTTCGCAG
14. GATCAAGTCC
15. GATCCAGTAC
16. GATCACGTAC
17. GATCTGACAC
18. GATCTCAGAC
19. GATCATAGCC
20. GATCAATCGC
21. GATCTAACCG
22. GATCGCATTG
23. GATCTGACTG
24. GATCATGGTC
25. GATCATAGCG
26. GATCTAAGGC

5. The tool of claim 1-4, wherein said 5'-oligonucleotide primers of claim 4 are used as 3'-oligonucleotide primers.

6. A process for application of the tool of claim 1 - 5, wherein total cell RNA is transcribed into cDNA, which is subsequently amplified with a certain number, preferentially 288 - 360, of parallel and independent polymerase chain reactions (PCR) by the tool. The resulting fragment pool of each reaction is separated on one lane of a nondenaturing polyacrylamide gel, the band pattern is determined and will be compared with a standard. If needed the DNA of the bands is isolated and used as a probe for gene isolation.

7. The process of claim 6, wherein each of said PCR reactions, preferentially a total number of 312, is separated on an appropriate number of polyacrylamide gels, preferentially 7 gels with 48 lanes. The band signals are detected by autoradiography or a corresponding nonradioactive proof, scanned and compared with the pattern of a reference cell.

8. The process of claim 6, wherein all of said PCR reactions of the reference cell are carried out with primers labeled with a certain flourescence dye and all of said PCR reactions of the investigated cell are carried out with primers, that are labeled with a different fluorescence dye.

9. The process of claim 6 and 8, wherein PCR fragments of the reference and investigated cell, which are labeled by primers of the same sequence but different dyes, are separated together in one lane of an automatic sequencer with an integrated dye-detection system and differences in gene expression are indicated by different dye signals at a specific position.

10. The process of claim 6, wherein a band is excised from the gel, extracted DNA is amplified and simultaneously biotin labeled by PCR and subsequently used for a hybridization with a denatured cDNA library and binding on streptavidin coated magnetic beads. After washing the beads, elution, bacteria transformation and plasmid preparation the specific bound cDNA is sequenced and identified as a known or unknown gene.

11. The process of claim 6,8 and 9, wherein the primers anneal at 40 °C during the PCR reaction.

## Revendications

1. Agent pour le diagnostic complexe de l'expression génique, caractérisé en ce qu'il est constitué d'au moins 288 paires d'oligonucléotides primers, chacune comportant une réserve d'au moins 24 5'-oligonucléotides primers ayant un nombre égal de G+C et A+T, et d'au moins 12 3'-oligonucléotides primers ayant une série de nucléotides T, de préférence de 8 à 12, et supplémentairement, au bout 3', dans 1 à 3 positions, toutes les combinaisons possibles des 4 nucléotides, qui sont couplées avec un marqueur approprié identifiable par un procédé de décèlement.

2. Agent pour le diagnostic complexe de l'expression génique selon la revendication 1, caractérisé en ce que tous les 5'-primers ou tous les 3'-primers sont marqués.

3. Agent pour le diagnostic complexe de l'expression génique selon les revendications 1 et 2, caractérisé en ce que, pour le marquage, il est fait usage de phosphore radioactif, d'un colorant fluorescent, de biotine ou d'un antigène.

4. Agent pour le diagnostic complexe de l'expression génique selon les revendications 1 à 3, caractérisé en ce que les 5'-oligonucléotides primers présentent les séquences suivantes:

1. 5' T A C A A C G A G G 3'
2. T G G A T T G G T C
3. C T T T C T A C C C
4. T T T T G G C T C C
5. G G A A C C A A T C
6. A A A C T C C G T C
7. T C G A T A C A G G
8. T G G T A A A G G G
9. T C G G T C A T A G

10. G G T A C T A A G G
11. T A C C T A A G C G
12. C T G C T T G A T G
13. G T T T T C G C A G
14. G A T C A A G T C C
15. G A T C C A G T A C
16. G A T C A C G T A C
17. G A T C T G A C A C
18. G A T C T C A G A C
19. G A T C A T A G C C
20. G A T C A A T C G C
21. G A T C T A A C C G
22. G A T C G C A T T G
23. G A T C T G A C T G
24. G A T C A T G G T C
25. G A T C A T A G C G
26. G A T C T A A G G C

5. Agent pour le diagnostic complexe de l'expression génique selon les revendications 1 et 4, caractérisé en ce que les 5'-oligonucléotides primers selon la revendication 4 sont utilisés comme 3'-oligonucléotides primers.

6. Méthode pour appliquer l'agent selon les revendications 1 à 5, caractérisée en ce que tout l'A.R.N. d'une cellule est transcrit en A.D.N.-c., qu'ensuite ce dernier est amplifié avec l'agent dans un certain nombre - de préférence de 288 à 360 - de réactions en chaîne de polymérase (réactions P.C.R.) parallèles et indépendantes, que le mélange de fragments ainsi obtenu dans chaque réaction est défait sur une voie d'un gel de polyacrylamide non dénaturé, que le dessin de bande est enregistré et comparé avec un dessin standard, que, le cas échéant, les A.D.N. sont isolés de certaines bandes et utilisés comme sonde pour l'isolation de gène.

7. Méthode selon la revendication 6, caractérisée en ce que chaque réaction P.C.R., de préférence d'un nombre total de 312, est conduite séparément sur le nombre correspondant de gels de polyacrylamide, de préférence 7 gels à 48 voies, que les signaux de bande sont visualisés par autoradiographie ou à l'aide de détecteurs non radioactifs appropriés, sont introduits par scanner ou comparés avec l'échantillon d'une cellule de référence.

8. Méthode selon la revendication 6, caractérisée en ce que toutes les réactions P.C.R. d'une cellule de référence sont effectuées avec des primers marqués avec un colorant fluorescent donné et que toutes les réactions P.C.R. de la cellule à examiner sont effectuées avec des primers marqués avec un colorant fluorescent différent.

9. Méthode selon les revendications 6 et 8, caractérisée en ce que les fragments de la cellule de référence et de la cellule à examiner, fragments marqués avec des primers de même séquence, mais avec des colorants différents, sont défaits en commun dans une voie d'un automate séquentiel équipé d'un système incorporé de détection de couleur et que les différences d'expression génique sont affichées par un signal de couleur différent dans une position donnée.

10. Méthode selon la revendication 6, caractérisée en ce qu'une bande est découpée du gène, que son A.D.N. est amplifié par P.C.R. et, en même temps, marqué avec de la biotine, qu'ensuite il est hybridé avec une bibliothèque A.D.N.-c. dénaturée et est fixé à des boules magnétiques de Streptavidin, et que le A.D.N.-c. spécifiquement fixé, après lavage des boules, élution, transformation des bactéries et préparation de plasmide, est séquencé pour être identifié comme gène connu ou gène inconnu.

11. Méthode selon les revendications 6, 8 et 9, caractérisée en ce que le processus d'"annealing" des primers dans la réaction P.C.R. s'effectue à 40 °C.
